# EUROPÄISCHE PATENTANMELDUNG

(11) **EP 1 138 990 A2**
(43) Veröffentlichungstag der Anmeldung: **04.10.2001**
(21) Anmeldenummer: 01107481.2
(22) Anmeldetag: 28.03.2001
(51) Int. Cl.: F16K 11/085, F16K 35/04

(54) **Mehrwegehahn**

(30) Priorität: 28.03.2000 DE 20005691 U
(71) Anmelder: B. Braun Melsungen AG, 34209 Melsungen (DE)
(72) Erfinder: Lotz, Norbert, 6182 Escholzmatt (CH); Siegmeier, Gunther, 6182 Escholzmatt (CH); Hayoz, Boris, 2545 Selzach (CH); Schlarb, Alois, Dr., 6110 Wolhusen (CH)
(74) Vertreter: Klingseisen, Franz, Dipl.-Ing.

(57) **Zusammenfassung**

Bei einem Mehrwegehaln mit einem hohlzylindrischen Gehäuse (1) mit Anschlußbohrungen (2,2',2'') auf dem Umfang und in dem Gehäuse drehbar gelagerten Küken (3) mit einem Durchgangskanal (6) quer zur Drehachse sind zur Markierung der einzelnen Öffnungsstellungen vorzugsweise an einem Bodenabschnitt (5) des Gehäuses axial abstehende Eingriffselemente (9) ausgebildet, die mit Rastnuten oder Rastvorsprüngen versehen sind, die mit Rastvorsprüngen oder Rastnuten auf dem Innenumfang des hohlzylindrischen Kükens (3) zusammenwirken.

## Beschreibung

Die Erfindung betrifft einen Mehrwegehahn, insbesondere einen Dreiwegehahn, für medizinische Geräte zum Fördern von Infusionslösungen, flüssigen Medikamenten und dergleichen.

Bei einem Mehrwegehahn kann das Hahnküken Zwischenstellungen einnehmen, in denen eine Leitung nicht vollständig abgesperrt bzw. eine Verbindung nicht vollständig offen ist.

Der Erfindung liegt die Aufgabe zugrunde, einen Mehrwegehahn so auszubilden, daß die Offen- und Schließstellungen des Hahnkükens exakt eingestellt werden können und das Hahnküken aus einer eingestellten Betriebsstellung nicht ohne weiteres verdreht werden kann.

Diese Aufgabe wird erfindungsgemäß im wesentlichen dadurch gelöst, daß zwischen Küken und Gehäuse in radialer Richtung wirkende Rasteinrichtungen vorgesehen werden, mittels denen die jeweilige Betriebsstellung des Kükens für die Bedienungsperson deutlich fühlbar vorgegeben und die jeweils eingenommene Betriebsstellung auch fixiert wird.

Die Erfindung wird beispielsweise anhand der Zeichnung näher erläutert. Es zeigen
- Fig. 1: eine perspektivische Ansicht eines Dreiwegehahns,
- Fig. 2: einen Längsschnitt durch eine erste Ausführungsform,
- Fig. 3: schematisch eine Querschnittsansicht längs der Linie A-A in Fig. 1,
- Fig. 4: einen Längsschnitt durch eine weitere Ausführungsform,
- Fig. 5: eine Querschnittsansicht längs der Linie B-B in Fig. 4,
- Fig. 6: einen Längsschnitt durch eine andere Ausführungsform,
- Fig. 6a: eine vergrößerte Darstellung des Eingriffsbereichs in Fig. 6 im Schnitt,
- Fig. 7: schematisch einen Querschnitt längs der Linie C-C in Fig. 6,
- Fig. 8: einen Längsschnitt durch eine weitere Ausführungsform,
- Fig. 9: einen Querschnitt längs der Linie D-D in Fig. 8, und
- Fig. 10: Ausführungsformen von Rippen bzw. Nocken und Rastnuten.

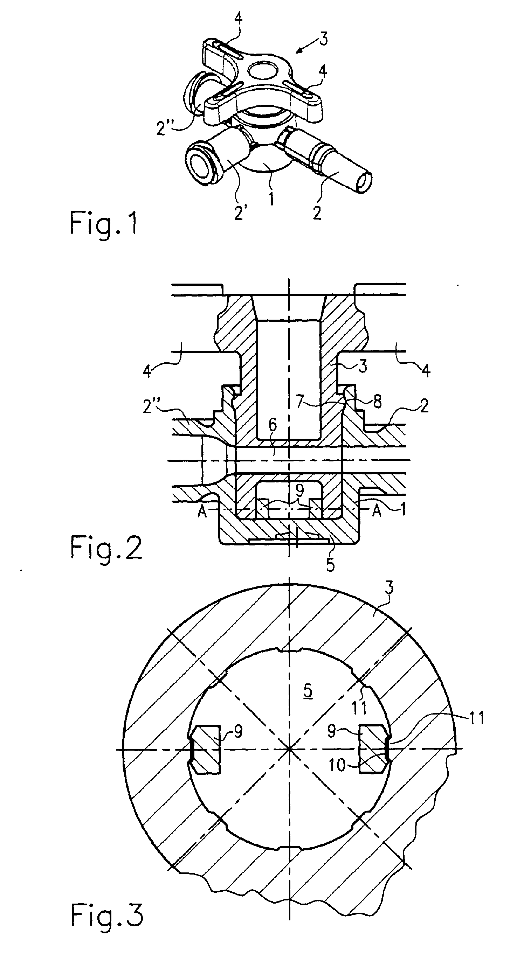

Fig. 1 zeigt einen Dreiwegehahn mit einem hohlzylindrischen Gehäuse 1, von dessen Umfang Leitungsstutzen 2, 2' und 2" radial abstehen, wobei die Leitungsstutzen 2' und 2" jeweils mit einem Luer-Kegel für den Anschluß einer Leitung versehen sind. Mit 3 ist ein in dem Gehäuse 1 verdrehbares Hahnküken bezeichnet, das drei um 90° versetzte Griff-Flügel 4 aufweist. Gehäuse und Küken sind aus Kunststoff gefertigt.

Fig. 2 zeigt im Längsschnitt eine Ausführungsform, bei der das hohlzylindrische Gehäuse 1 einen geschlossenen Bogen 5 aufweist, auf dem die Stirnseite des hohlzylindrischen Kükens 3 anliegt, durch das quer zur Drehrichtung ein Durchgangskanal 6 führt, der mit den Leitungsstutzen 2 bis 2" entsprechenden Gehäusebohrungen ausrichtbar ist. Das Küken 3 weist im oberen Abschnitt einen Wulst 7 auf dem Außenumfang auf, der in eine Ringnut 8 auf dem Innenumfang des Gehäuses 1 eingreift und das Küken 3 im Gehäuse 1 in Achsrichtung hält. Um den Durchgangskanal 6 liegt das Küken mit dem Außenumfang am Innenumfang des Gehäuses 1 abdichtend an.

Auf der Innenseite des Gehäusebodens 5 sind zwei axial abstehende Zapfen 9 an diametral gegenüberliegenden Stellen angeformt, die auf der Außenseite mit einer axial verlaufenden flachen Nut 10 versehen sind, wie Fig. 3 zeigt, in die auf dem Innenumfang des Kükens 3 am unteren Randabschnitt ausgebildete Rippen bzw. Nocken 11 einrasten können.

Es können auch mehrere axial abstehende Zapfen 9 am Gehäuseboden 5 ausgebildet sein, und ebenso ist es möglich, an den Zapfen 9 eine vorstehende Rippe 11 vorzusehen, der in eine entsprechende Ausnehmung bzw. Nut 10 auf dem Innenumfang des Kükens einrastet.

Vorzugsweise werden in einem Winkelabstand von 45° Rippen bzw. Nocken 11 oder entsprechende Nuten 10 auf dem Umfang des Kükens 3 vorgesehen.

Durch die in radialer Richtung vorstehenden Rippen 11 und die in radialer Richtung elastischen Zapfen 9 wird beim Verdrehen des Kükens die Abdichtung zwischen Küken und Gehäuse sowie der Sitz des Kükens 3 im Gehäuse 1 mittels des Wulstes 7 durch die Rasteinrichtung nicht beeinträchtigt.

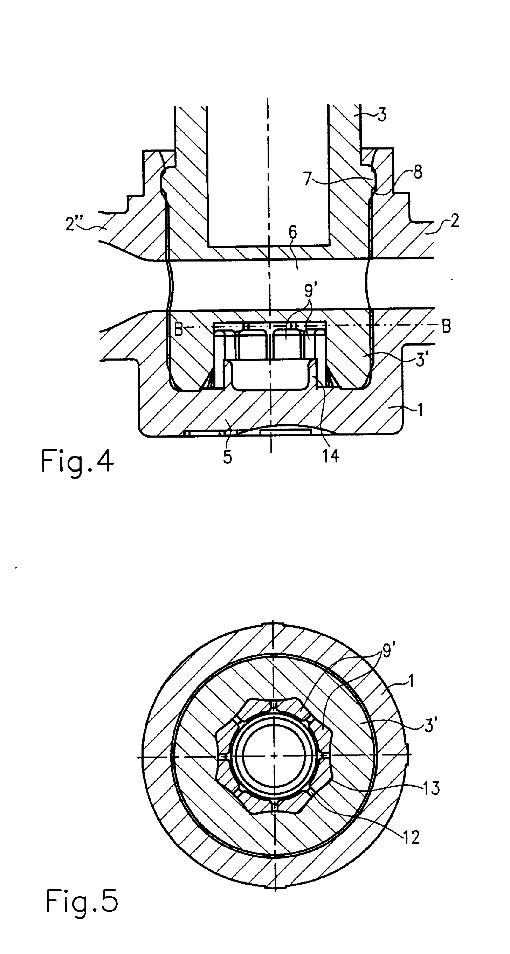

Fig. 4 und 5 zeigen eine Ausführungsform, bei der am Gehäuseboden 5 insgesamt acht ringförmig angeordnete Zapfen 9' so angeformt sind, daß sie einen geringen Abstand 12 voneinander haben und in radialer Richtung elastisch beweglich sind. Die einzelnen Zapfen 9' weisen auf der radial außen liegenden Seite eine Rastrippe 13 mit flachen Seitenflanken auf, die an die Seitenflanken des benachbarten Zapfens anschließen.

Wie der Längsschnitt in Fig. 4 zeigt, ist am Gehäuseboden 5 auf dem Innenumfang der Zapfen 9' ein Ringkörper 14 angeformt, der sich etwa über die halbe Höhe der Zapfen 9' erstreckt und diese in radialer Richtung abstützt.

Der untere Abschnitt 3' des Kükens 3 weist auf dem Innenumfang eine der Form der Zapfen 9'komplementäre Formgebung auf. Der Innenumfang des Kükenabschnittes 3' ist in etwa achteckig ausgebildet, wobei die acht Seitenflächen etwas radial nach innen gewölbt und die Übergangsstellen zwischen den Seitenflächen abgerundet sind, entsprechend der Form der Rastrippen 13 an den Außenseiten der Zapfen 9' und deren leicht gebogenen Seitenflanken.

Bei der Ausführungsform nach den Fig. 4 und 5 können auch die radial nach innen gewölbten Seitenflächen am Küken 3 als Vorsprünge und die entsprechende Vertiefung zwischen benachbarten Rastrippen 13 als Nut bezeichnet werden.

Beim Verdrehen des Kükens 3 wird auf die Dichtfläche zwischen Küken und Gehäuse ein Druck ausgeübt, so daß die Abdichtung nicht beeinträchtigt wird.

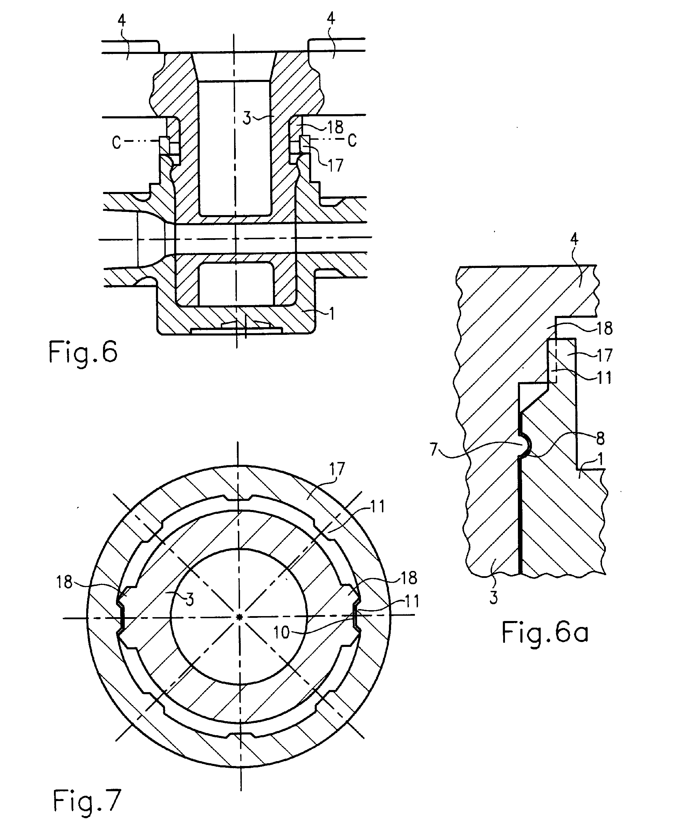

Fig. 6 und 7 zeigen eine weitere Ausführungsform, bei der am oberen Rand des Gehäuses 1 ein Ringkörper 17 angeformt ist, auf dessen Innenumfang Nocken oder Rippen 11 in einem Winkelabstand von 45° ausgebildet sind, die mit Nuten 10 an diametral gegenüberliegenden Vorsprüngen 18 in Rasteingriff treten, die aber Küken 3 im Übergangsbereich zwischen Kükenkörper und radial abstehenden Griff-Flügeln 4 angeformt sind.

Der Ringkörper 17 kann entsprechend der Ausgestaltung in Fig. 2 und 3 in einzelne Segmente unterteilt ausgebildet sein, wobei jedes Segment mit einem Nocken bzw. einer Rippe 11 versehen ist.

Wie die schematische Schnittansicht in Fig. 6a zeigt, hat der mit den Nocken 11 versehene Ringkörper 17 eine geringere Wandstärke als der angrenzende Wandabschnitt des Gehäuses 1. Auf diese Weise wird gewährleistet, dass beim Verdrehen des Kükens die Halterung am Wulst 7 nicht beeinträchtigt wird.

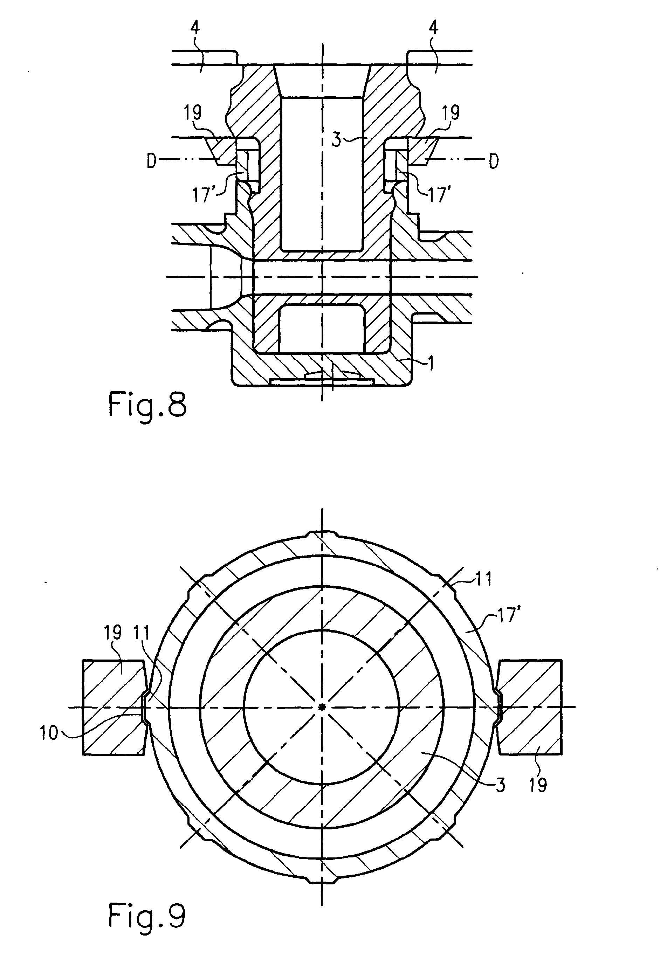

Bei der Ausführungsform nach den Fig. 8 und 9 sind auf dem Außenumfang des am oberen Rand des Gehäuses 1 angeformten Ringkörpers 17' Nocken oder Rippen 11 ausgebildet, die in Nuten 10 an zwei diametral gegenüberliegenden Vorsprüngen 19 einrasten, welche an den Unterseiten der Griff-Flügel 4 des Kükens 3 angeformt sind. Bei dieser Ausgestaltung treten am Ringkörper 17' beim Verdrehen des Kükens radial nach innen gerichtete Kräfte auf, so daß die Halterung des Kükens am Wulst 7 im Gehäuse nicht beeinträchtigt wird.

Bei dieser Ausführungsform nach den Fig. 8 und 9 kann der dem oberen Rand 17' des Gehäuses 1 entsprechende Ringkörper 17' einen größeren Durchmesser haben als das Gehäuse, wobei die Vorsprünge 19 an den Griff-Flügeln 4 weiter radial außen angeformt werden können. Hierdurch greift ein größeres Drehmoment beim Verdrehen des Hahnkükens an den Rasteinrichtungen an, wodurch die Kükeneinstellung erleichtert wird.

Es kann auch am dritten Griff-Flügel 4 ein entsprechender Vorsprung 19 für den Rasteingriff mit einem der Nocken 11 ausgebildet sein.

Die Ausführungsformen nach den Fig. 6 bis 9 sind für einen Mehrwegehahn ohne Gehäuseboden 5, also mit unten offenem Gehäuse 1 geeignet.

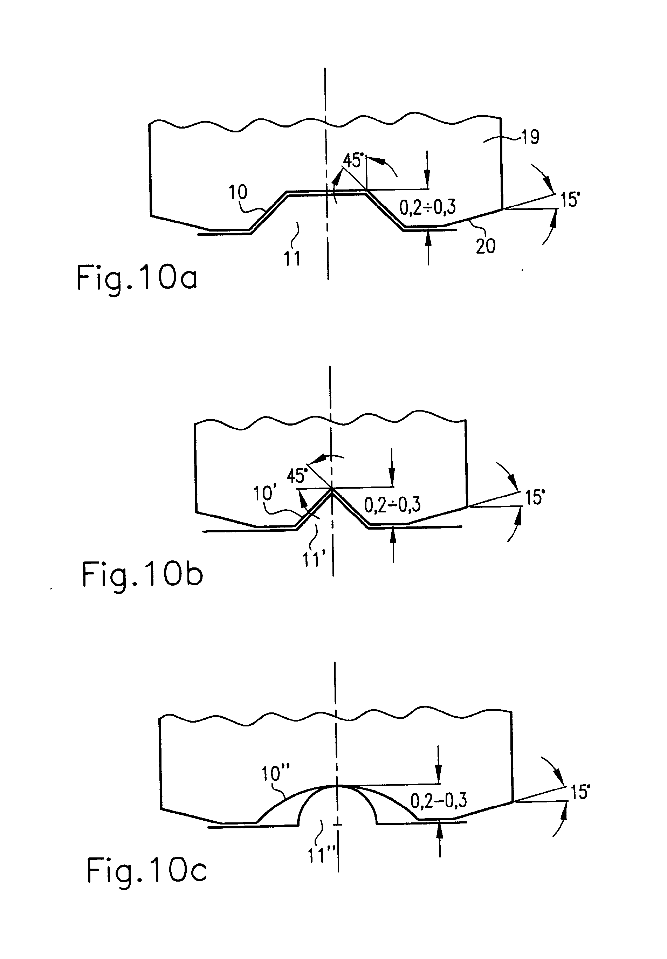

Fig. 10 zeigt verschiedene Rippen- bzw. Nockenformen, wobei Fig. 10 a die in den vorausgehend beschriebenen Ausführungsformen verwendete Nockenform im einzelnen wiedergibt. Der Vorsprung 19, in dem die Nut 10 ausgebildet ist, weist eine abgeschrägte Anlauffläche 20 auf, die in einem Winkel von ca. 15° zur Horizontalen in Fig. 10a liegt, während die Seitenflanken der Rippe bzw. des Nockens 11 und entsprechend die Seitenflächen der Nut 10 einen Winkel von ca. 45° bilden.

Fig. 10b zeigt eine im Querschnitt dreieckige, in Achsrichtung verlaufende Rippe 11', die in eine komplementär geformte Nut 10' eingreift. Bei dieser Ausführungsform entspricht die Höhe der Rippe 11' bzw. die Tiefe der Nut 10' etwa 0,2 bis 0,3 mm wie bei der Ausführungsform in Fig. 10a, wobei die Seitenflanken der Rippe 11' eine Neigung von 45° haben.

Fig, 10 c zeigt eine im Querschnitt etwa halbkreisförmige Rippe 11", die in eine konkave Nut 10" eingreift, deren Wölbung mit einem größeren Radius ausgebildet ist als die Rundung der Rippe 11".

Anstelle der in den Fig. 10a und b wiedergegebenen Schrägflächen können auch abgerundete Flächenabschnitte vorgesehen werden, um den Einrastvorgang weicher zu gestalten.

Es sind verschiedene Abwandlungen der beschriebenen Bauformen möglich. So können beispielsweise anstelle eines geschlossenen Bodens 5 des Gehäuses 1 sternförmig angeordnete Stege vorgesehen werden, die vom Umfang des Gehäuses 1 radial nach innen verlaufen, wobei an diesen Stegen die für die Verrastung vorgesehenen Zapfen 9 angeformt sein können.

Bei der Ausführungsform nach den Fig. 2 und 3 ist es beispielsweise auch möglich, in der Mitte des Bodens 5 zwischen den Zapfen 9 eine Bohrung vorzusehen, so daß der Boden des Gehäuses offen ist. Die Zapfen 9 sind hierbei auf einem Ringbereich um die Bohrung angeformt. Eine solche Bohrung ist auch bei den Ausführungsformen der Fig. 4, 6 und 8 möglich.

Nach einer weiteren Ausgestaltung kann bei der Bauform nach den Fig. 8 und 9 anstelle der Vorsprünge 19 ein nicht dargestellter Ringkörper an der Unterseite der Griff-Flügel 4 angeformt oder in anderer Weise befestigt sein, der den am Gehäuse angeformten Ringkörper 17' übergreift, wie die Schnittdarstellung in Fig. 8 zeigt. Bei einer solchen Ausführungsform umschließt der an den Griff-Flügeln 4 angebrachte Ringkörper in der Querschnittsansicht nach Fig. 9 den Ringkörper 17', wobei während eines Rastvorgangs bzw. einer Relativverdrehung zwischen den beiden Ringkörpern über den Umfang verteilt Kräfte radial nach innen auf das Gehäuse wirken. Bei der Ausführungsform nach Fig. 9 wirken bei einer Relativverdrehung zwischen Vorsprüngen 19 und Ringkörper 17' radial nach innen gerichtete Kräfte nur an diametral gegenüberliegenden Stellen.

## Patentansprüche

1. Mehrwegehahn, umfassend
ein etwa hohlzylindrisches Gehäuse (1) mit Anschlußbohrungen auf dem Umfang und
ein in dem Gehäuse drehbar gelagertes Küken (3) mit einem Durchgangskanal (6) quer zur Drehachse, der mit den Anschlußbohrungen im Gehäuse ausrichtbar ist, wobei an einem Bodenabschnitt des Gehäuses (1) axial abstehende Eingriffselemente (9,9') ausgebildet sind, die mit Rastnuten (10) oder Rastvorsprüngen (11) versehen sind, die mit Rastvorsprüngen oder Rastnuten auf dem Innenumfang des hohlzylindrisch ausgebildeten Kükens (3) zusammenwirken.

2. Mehrwegehahn nach Anspruch 1,
wobei die Eingriffselemente als einander diametral gegenüberliegende Zapfen (9) ausgebildet sind.

3. Mehrwegehahn nach Anspruch 1,
wobei die Eingriffselemente als Zapfen (9') ausgebildet sind, die ringförmig angeordnet sind.

4. Mehrwegehahn nach Anspruch 3,
wobei der Innenumfang des Kükens (3) etwa achtseitig ausgebildet ist und die einzelnen Seitenflächen leicht nach innen gewölbt ausgebildet sind, während die Außenfläche der ringförmig angeordneten Zapfen (9') komplementär mit dem Innenumfang des Kükens (3) gestaltet ist.

5. Mehrwegehahn nach den Ansprüchen 3 und 4,
wobei die in radialer Richtung elastisch bewegbaren Zapfen (9') durch einen innerhalb der ringförmig angeordneten Zapfen angeformten Ringkörper (14) abgestützt sind.

6. Mehrweghahn nach den vorhergehenden Ansprüchen, wobei das Gehäuse (1) unten einen geschlossenen Boden (5) aufweist.

7. Mehrwegehahn, umfassend
ein etwa hohlzylindrisches Gehäuse mit Anschlußbohrungen auf dem Umfang und ein in dem Gehäuse drehbar gelagertes Küken (3) mit einem Durchgangskanal (6) quer zur Drehachse, der mit den Anschlußbohrungen im Gehäuse ausrichtbar ist, wobei am oberen Rand des Gehäuses (1) ein mit Rastvorsprüngen (11) oder Rastnuten (10) versehener Ringkörper (17) ausgebildet ist, der mit Rastelementen zusammenwirkt, die auf der Unterseite von Griff-Flügeln (4) des Kükens (3) angeordnet sind, wobei diese Rastelemente Rastnuten oder Rastvorsprünge aufweisen.

8. Mehrwegehahn nach Anspruch 7, wobei auf der Unterseite der Griff-Flügel (4) ein Ringkörper ausgebildet ist, der auf der radial innenliegenden Seite Rastnuten (10) oder Rastvorsprünge (11) aufweist, die mit Rastvorsprüngen (11) oder Rastnuten (10) auf dem Außenumfang des oberen Randes (17') des Gehäuses (1) zur Ausbildung einer Rasteinrichtung zusammenwirken.

9. Mehrwegehahn nach Anspruch 7,
wobei auf der Unterseite von diametral gegenüberliegenden Griff-Flügeln (4) ein Vorsprung (19) angeformt ist, der auf der radial innenliegenden Seite eine Rastnut (10) oder eine Rastrippe (11) aufweist, die mit Rastrippen (11) oder Rastnuten (10) auf dem Außenumfang des oberen Randes (17') des Gehäuses (1) zur Ausbildung einer Rasteinrichtung zusammenwirken.

10. Mehrwegehahn nach Anspruch 7,
wobei auf dem Innenumfang des oberen Randes (17) des Gehäuses (1) Rastrippen (11) oder Rastnuten (10) ausgebildet sind, die mit Rastnuten (10) oder Rastrippen (11) am Außenumfang des Kükens (3) zusammenwirken.

11. Mehrwegehahn nach Anspruch 10,
wobei an diametral gegenüberliegenden Stellen des Kükens (3) Vorsprünge (18) mit einer Nut (10) ausgebildet sind.

12. Mehrwegehahn nach den vorhergehenden Ansprüchen,
wobei in einem Winkelabstand von 45° Raststellungen ausgebildet sind.
